(19) **Europäisches Patentamt**

**European Patent Office**

**Office européen des brevets**

(11) **EP 1 465 089 A2**

(12) **EUROPEAN PATENT APPLICATION**

(43) Date of publication:
06.10.2004 Bulletin 2004/41

(51) Int Cl.7: **G06F 17/50**

(21) Application number: 04008047.5

(22) Date of filing: 02.04.2004

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: 03.04.2003 US 460832 P

(71) Applicant: **Accelrys Inc.**
San Diego, CA 92121 (US)

(72) Inventors:
• **Sykes, Paul**
  **Girton Cambridge, CB3 0PG (GB)**
• **Parkin, Richard**
  **Cambridge, CB4 3JD (GB)**

(74) Representative:
**Sternagel, Fleischer, Godemeyer & Partner Patentanwälte**
**Braunsberger Feld 29**
**51429 Bergisch Gladbach (DE)**

(54) **Method and system for atom matching for reactant and product atomic and molecular systems**

(57) The present invention refers to methods and systems for matching atoms in a system of reactant atoms with atoms in a system of product atoms, wherein the matching is accomplished by a chain of matching algorithms that may include unique atom matching, matching regions of common topology, and matching atoms based on similarity of geometry.

## Description

The present invention relates to molecular modeling, particularly of a reaction trajectory. More specifically, the present invention relates matching of atoms in a reactant system with atoms in a product system. Disclosed herein are methods and systems for matching atoms in a system of reactant atoms with atoms in a system of product atoms. The matching is accomplished by a chain of matching algorithms that may include unique atom matching, matching regions of common topology, and matching atoms based on similarity of geometry. Centers of common topology may be chosen by using unique atoms or user defined atoms as seeds for the topology matching algorithm. The topology matching algorithm may iteratively match atoms with regions of common topology. Geometric algorithms may be used to match any remaining ambiguous atoms. A library of geometric algorithms may be provided such that the one providing the best performance may be chosen, either automatically or by user input.

In computer modeling of chemical reactions, a system of atoms changes from a reactant configuration to a product configuration. For many methods of modeling such reaction transitions (for example, using Linear Synchronous Transit as described in Halgren and Lipscomb Chem. Phys. Lett. 49, 225 (1977)), it is first necessary to have established a complete match between corresponding atoms in the reactant and product structures. Many current methods require users to identify these corresponding atoms by hand, for example by entering two sets of atomic Cartesian coordinates in the proper order.

Lynch and Willett (J. Chem. Inf. Comput. Sci. 18(3), 154 (1978)) originally demonstrated how application of an existing technique for assigning unique codes to molecules based on the connectivity of contained atoms (Morgan, J. Chem. Doc. 5, 107 (1965)) could be used for matching equivalent atoms between molecules. In the Lynch and Willett paper, a matching procedure is described which identifies common substructures and by an iterative process that attempts to match equivalent atoms. Although this can provide an automated match of molecular fragments in some cases, it is usually the case that ambiguous fragments remain unresolved.

Thus, it is an object of the present invention to provide an automated procedure for matching atoms that can match all atoms in a reactant system of atoms with all atoms in a product system of atoms with no or relatively little user input.

This object is met by a computer-implemented method of matching atoms in a reactant system of atoms with corresponding atoms in a product system of atoms, said reactant system producing said product system in a chemical reaction, said method comprising:

a) matching at least one group of one or more atoms in said reactant system with at least one group of one or more atoms in said product system if said groups of one or more atoms are within regions of common topology; and

b) matching a plurality of other atoms in said reactant system with a plurality of other atoms in said product system based on similarity in geometry of said other atoms in said reactant system with geometry of said other atoms in said product system.

Disclosed herein is a computer-implemented method of matching atoms in a reactant system of atoms with corresponding atoms in a product system of atoms, the reactant system producing the product system in a chemical reaction, the method comprising: matching at least one group of one or more atoms in the reactant system with at least one group of one or more atoms in the product system if the groups of one or more atoms are within regions of common topology; and matching a plurality of other atoms in the reactant system with a plurality of other atoms in the product system based on similarity in geometry of the other atoms in the reactant system with geometry of the other atoms in the product system.

Also disclosed herein is a computer-implemented method of matching all atoms in a reactant system of atoms with corresponding atoms in a product system of atoms, the reactant system producing the product system in a chemical reaction, the method comprising: a) receiving data representing atom arrangement and bonding in the reactant and product systems; b) matching a group of one or more atoms in the reactant system with a group of one or more atoms in the product system if the groups are within regions of common topology; c) repeating step b until no more groups within a region of common topology are found; and d) matching remaining unmatched atoms based on similarity in geometry of the remaining atoms in the reactant system with geometry of the remaining atoms in the product system.

Also disclosed herein is a computer-implemented system for matching atoms in a reactant system of atoms with corresponding atoms in a product system of atoms, the reactant system producing the product system in a chemical reaction, the system comprising: a memory storing data representing atom arrangement and bonding in the reactant and product systems; a topology matching module operative to match a group of one or more atoms in the reactant system with a group of one or more atoms in the product system if the groups are within regions of common topology; and a geometry matching module operative to match atoms based on similarity in geometry of atoms in the reactant system with geometry of atoms in the product system.

In some embodiments, the present invention provides methods and systems for matching atoms in a reactant system of atoms with corresponding atoms in a product system of atoms. The systems of atoms may consist of one or more individual atoms, one or more molecules, and/or atoms that are part of a solid substrate. The same atoms are present in the reactant and product systems of atoms, however, generally the atom arrangement and bonding will be altered. The atom arrangement and bonding of the product system of atoms is produced by a chemical reaction involving the reactant system of atoms. In some embodiments, the atom arrangement and bonding in the reactant and product systems of atoms will have been characterized or modeled prior to matching.

A new atom matching procedure has been developed which requires no or relatively little user input. This procedure consists of a chain of various matching algorithms. By chaining together multiple algorithms, the number of atoms automatically matched can be increased over matches obtained using any one of the algorithms. In some embodiments, all of the atoms in the reactant and product system of atoms can be matched for a wide variety of reactant/product systems with no user intervention, even when large numbers of atoms are involved.

There are three basic components to the procedure. First, initial 'definite' matches between atoms are established based either on user intervention or by using certain unique atom property algorithms. Secondly, matches may be made using a common topology search, such as an implementation of the Lynch-Willett procedure. Such common topology searches may use any initial definite matches as 'seeds' for the search. Finally, remaining ambiguous atoms may be matched using one or more geometric algorithms.

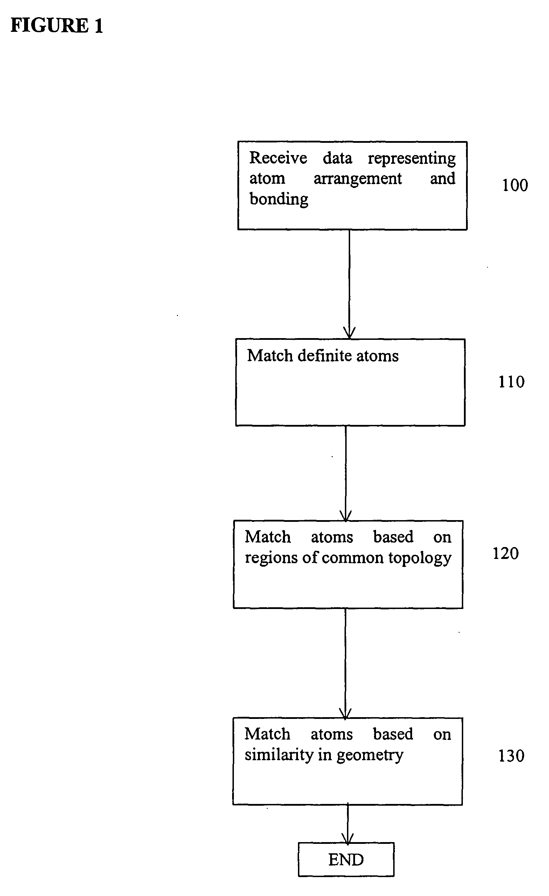

In one embodiment, depicted in the flow chart of Figure 1, a method is provided for matching atoms. At block 100, data representing the atom arrangement and bonding of the reactant and product systems of atoms are received. Next, at block 110, initial definite atoms are matched. At block 120, groups of atoms in the reactant and product systems are matched if they are within a region of common topology. Each group of atoms may include one or more atoms. In some embodiments, the region of common topology is larger than the group of atoms matched. In some embodiments, multiple groups of atoms are matched by finding multiple regions of common topology. Generally, it is advantageous to remove the atoms within a matched group from consideration before finding other groups of matched atoms based on topology. At block 130, at least some of the atoms not matched as unique atoms or by the common topology algorithm are matched based on similarity in the geometry of the atoms in the reactant system with the geometry of the atoms in the product system. Any of a number of geometric algorithms may be used to determine similarity in geometry. In some embodiments, the geometric algorithm is user selected. In other embodiments, the geometric algorithm is automatically selected depending on the characteristics of the atoms to be matched. It should be recognized that not all algorithms in the chain of algorithms depicted in Figure 1 might be necessary. For example, there may be no definite atoms matched. In such a case, matching will begin at block 120 by matching regions of common topology.

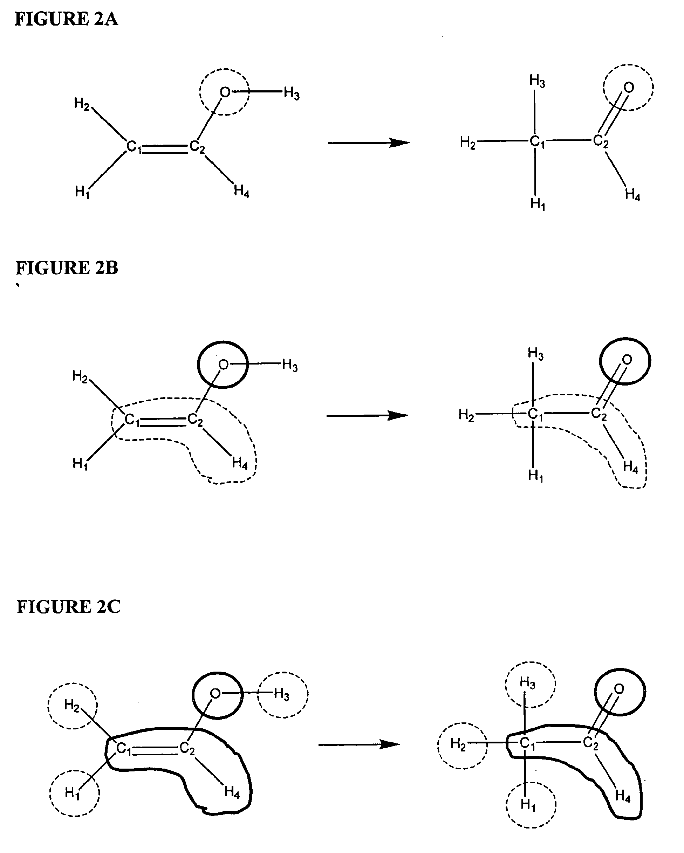

An example of the implementation of the procedure of Figure 1 is illustrated in Figures 2A to 2C. These figures depict the simple tautomerization reaction of ethenol being converted to acetaldehyde. The goal is to match atoms in the reactant ethenol with the corresponding atoms in the reactant acetaldehyde. In Figure 2A, corresponding to block 110 in Figure 1, a definite atom match is made between the oxygen atoms. Such a match is possible because there is only one oxygen atom each in the reactant and product molecules. In Figure 2B, corresponding to block 120 in Figure 1, a subset of the remaining unmatched atoms are matched based on a region of common topology. In this case, it is recognized that the atom subgroup $C_1$, $C_2$, and $H_4$ in the reactant and product molecules have a similar topology and can thus be matched. In Figure 2C, corresponding to block 130 in Figure 1, the remaining unmatched atoms, $H_1$, $H_2$, and $H_3$ may be matched using a geometric algorithm. For example, the algorithm may match $H_1$ in the product molecule to $H_1$ in the reactant molecule, $H_2$ in the product molecule to $H_2$ in the reactant molecule, and $H_3$ in the product molecule to $H_3$ in the reactant molecule based on a computation that the match results in the least amount of total relative movement of the respective atoms.

### Definite Atom Matching

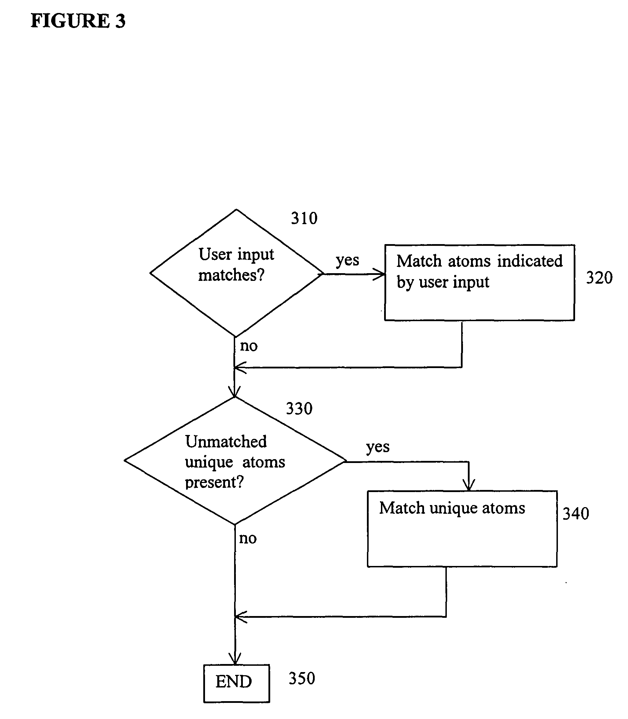

Figure 3 depicts one embodiment of a definite atom matching procedure. This embodiment identifies definite atoms based both on any user-identified matches as well as unique atoms. Starting with decision block 310, it is determined if a user has provided input identifying any definite atoms. If so, those atoms are matched at block 320. Next, decision block 330 determines whether there are any unique atoms present in the remaining unmatched atoms. If unique atoms are detected, those atoms are matched without the need of user input at block 340. For example, the reactant and product systems may contain only one oxygen atom and one nitrogen atom. In this case, a definite match can be made between the reactant oxygen and nitrogen to the product oxygen and nitrogen. In some embodiments, the initial definite matches made at blocks 320 and 340 are used as 'seeds' for common topology matching. This procedure is defined in more detail below.

It should be recognized that in some embodiments, definite matching is accomplished without any user input. Thus, methods and systems can be provided that minimize or eliminate the need for user intervention with an automated matching chain of algorithms.

### Common Topology Matching

The topology surrounding a given atom may be characterized by considering several properties including connectivity values, the types of atoms surrounding the atom, and bond patterns. The topological region around a given atom may be extended to any size. In this way, a common topological region containing several atoms may be found and the atoms matched. For example, the nth-order topology surrounding an atom may be determined by considering atoms to a radius of (n-1) bonds from an atom. In some embodiments, only atoms within a radius smaller than the radius of common topology are matched in order to reduce the occurrence of false matching. For example, for a common nth-order topology surrounding a central atom, only atoms within a radius of (n-2) bonds are matched.

In some embodiments, atoms matched in an initial definite match are used as a central starting point (seeds) for finding regions of common topography. In such cases, the regions of common topography will be centered on the initial definite match atoms. In other embodiments, centers of common topology are found automatically. Such a mode is useful if no seeds were identified or all seeds have already been used to find regions of common topography.

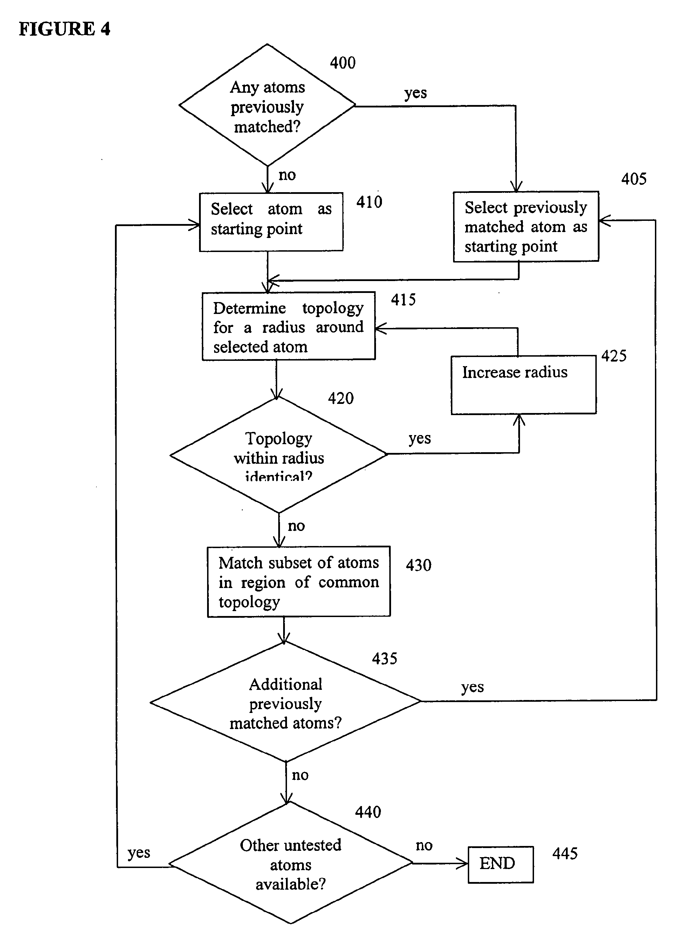

One embodiment of a topographical matching algorithm is depicted in Figure 4. At decision block 400, it is determined whether any initial definite match atoms have been identified. If so, one of these atoms is selected to be the center starting point of a topology search in block 405. If there are no initial definite match atoms, another atom may be selected as the starting point at block 410. At block 415, the topology surrounding the central atom is determined to a specified radius in both the reactant system of atoms and the product system of atoms. At decision block 420, it is determined if the topologies of the reactant system and product system within the radius are identical. If so, the radius for topological determination is increased at block 425, and the topology is recalculated at block 415. This process is repeated until increasing the radius produces regions that are topological distinct. At that point, at least some of the atoms within the regions of common topology are matched at block 430. As discussed above, the atoms matched may be located in a radius smaller than the region of common topology and thus be a subset of the atoms within the region of common topology. The matched atoms are removed from consideration in further matching and topology determinations. At decision block 435, it is determined if there are any further initial definite match atoms that have not been removed from consideration. If so, the procedure returns to block 405 to find additional common topological regions. If no other initial definite match atoms are available, decision block 440 determines whether other atoms are available to use as central starting points for topology determination. If so, the procedure returns to block 410 for further operation. If not, the procedure ends at block 445.

In some embodiments, the topology surrounding an atom is characterized by its nth-order connectivity value as defined in the Lynch-Willett procedure. In this procedure, the nth-order connectivity value, $V_i^n$, is given by:

$$V_i^n = 2V_i^{n-1} + \Sigma V_j^{n-1}$$

where the summation over $j$ is over all atoms adjacent to atom $i$.

Geometric Similarity Matching

For the remaining atoms or atom groups that remain ambiguous, one or more geometrical algorithms is advantageously made available to estimate a match for any remaining unmatched atoms. In some embodiments, a library of geometric algorithms is available from which an appropriate algorithm may be chosen based on performance (by looking at size of subgroup) and applicability (by looking at geometrical dispersal of atoms). For example, for small groups of ambiguous atoms, a match may be made between N reactant atoms and N product atoms by analyzing each of the different possible matches (N! possible matches if all N atoms are the same), and choosing the match that minimizes the total deviation in relative distances/orientation between the atoms of the group in the reactant and product states. Because computational cost of this procedure is of the order N!, this process is advantageously used only for relatively small groups of ambiguous atoms. In one embodiment of the invention, this procedure is used for groups of about six or less. If there is a larger group of ambiguous atoms, these can be broken up into smaller groups, or a different algorithm may be selected (preferably automatically) that chooses ambiguous reactant atoms individually and matches them one at a time to atoms of the same type in the product system that are closest to their original position.

In some embodiments, a user is allowed to select which geometric algorithm(s) are to be used. In other embodiments, the geometric algorithm(s) are chosen automatically. In some embodiments, more than one geometric algorithm will be used. For example, after initial definite matching and topological matching, there may be several groups of atoms that remain unmatched. It may be advantageous to first use an algorithm that matches groups of reactant atoms with corresponding groups of product atoms. Atoms within the individual groups can then be matched using an appropriate geometric algorithm. As discussed above, groups containing only a few atoms may be best matched using a different algorithm than groups containing many atoms.

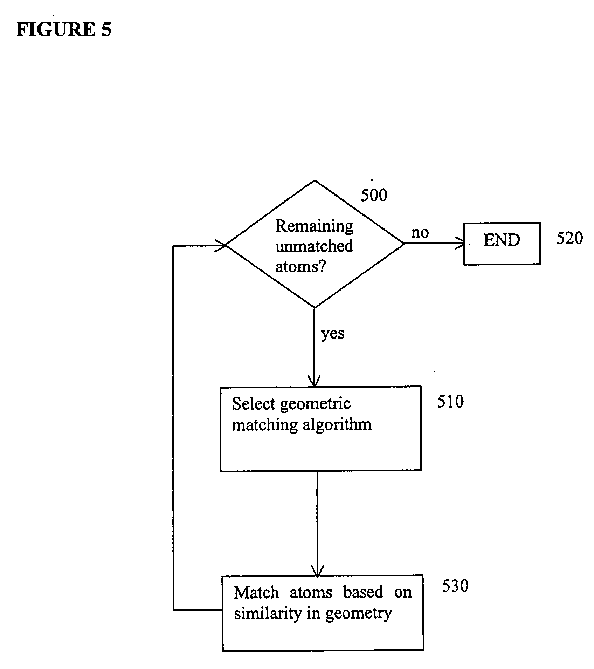

One embodiment of a geometric matching algorithm is depicted in Figure 5. At decision block 500, it is determined if there are any remaining unmatched atoms. If so, a geometric matching algorithm is selected at block 510. If there are no remaining unmatched atoms, the procedure ends at block 520. At block 530, the selected geometric matching algorithm is applied to one or more groups of atoms to obtain possible matches. Not all remaining atoms may be matched at block 530 if the selected geometric matching algorithm is not appropriate for all groups of remaining atoms. Thus, the procedure then returns to decision block 510 to determine if there are remaining unmatched atoms. If so, a different geometric matching algorithm appropriate for one or more remaining groups of atoms can be selected at block 510 and applied at block 530. The procedure can be repeated until there are no remaining unmatched atoms.

Computer-Implemented System

In one embodiment, a computer-implemented system is provided having atom matching functionality. In some embodiments, the system comprises at least a memory for storing data representing atom arrangement and bonding in the reactant and product atom systems, a unique atom matching module operative to match unique atoms, a topology matching module operative to match atoms based on common topology, and a geometry matching module operative to match atoms based on similarity in geometry. The unique atom matching module receives atom data from the memory and proceeds to match unique atoms. The topology matching module then receives atom data from memory and executes an algorithm, such as that described above, to find regions of common topology and then match groups of one or more atoms within the regions of common topology. The geometry matching module can also receive atom data from the memory to match atoms using one or more geometric algorithms.

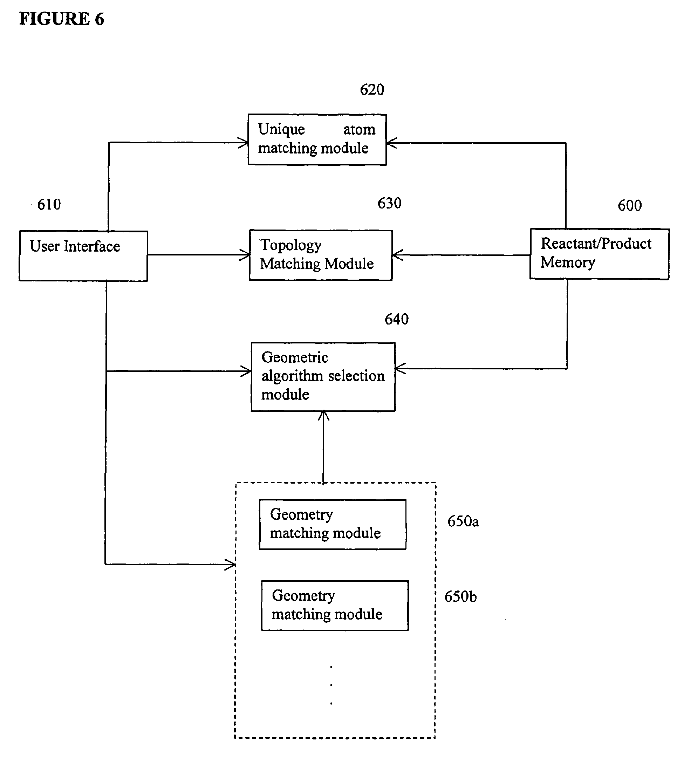

One embodiment of a computer-implemented system is depicted in Figure 6. The system comprises a memory 600, a user interface 610, a unique atom matching module 620, a topology matching module 630, a geometric algorithm selection module 640, and one or more geometry matching modules 650a and 650b. The memory 600 stores data representing atom arrangement and bonding in the reactant and product systems of atoms. Operation of the system may be such that the unique atom matching module 620 receives reactant and product atom data from memory 600 to determine whether there are any atoms that are unique and to match such atoms. User interface 610 may receive input from a user to manually match any atoms. The topology matching module 630 receives the atoms matched by the unique atom matching module 620 and by user interface 610 to use as seeds in common topology searching. The topology matching module 630 uses atom data in memory 600 to iteratively calculate regions of common topology, removing matched groups of atoms from further calculations as they are found. Once no more regions of common topology are found, the geometric algorithm selection module 640 receives the remaining unmatched atoms. In some embodiments, the geometric algorithm selection module 640 automatically selects one or more geometry matching modules 650a and 650b to operate on the unmatched atoms by considering the number and/or distribution of remaining atoms. In some embodiments, the geometric algorithm selection module 640 makes its selection based on user input received from user interface 610. The selected geometry matching modules 650a and 650b are used to match the remaining unmatched atoms. The unique atom matching module 620, topology matching module 630 and geometry matching modules 650a and 650b output the resulting atom matches to user interface 610 so that a user can view the matches. In some embodiments, the user interface 610 also allows the user to modify the resulting matches.

In various embodiments, not all components of the system of Figure 6 need be present in order to provide a functioning system. For example, in some embodiments the user interface 610 and unique atom matching module 620 may be absent or the geometric algorithm selection module 640 may be absent.

It will be appreciated that the system may be embodied in a general purpose computer having or being connected to a computer readable medium such as disk, CD, electronic memory, or the like that stores software code causing the computer to perform the steps outlined above. Computer readable media will also store data representing reactant and product atom arrangement and bonding, the library of atom matching algorithms, and the system will retrieve this information as necessary to perform the above described functions. A display and user input device such as a keyboard and/or mouse may also be provided to implement the above described user input and user interface.

FIGURE 1 depicts a flowchart illustrating a chain of atom matching algorithms including definite atom matching, topology matching, and matching based on similarity in geometry.

FIGURE 2 illustrates the operation of the chain of matching algorithms depicted in Figure 1 on a reactant and product molecule.

FIGURE 3 depicts a flowchart illustrating a definite atom matching algorithm.

FIGURE 4 depicts a flowchart illustrating an algorithm for atom matching based on common topological regions.

FIGURE 5 depicts a flowchart illustrating an algorithm for matching atoms based on similarity in geometry.

FIGURE 6 illustrates a computer-implemented system for atom matching.

**Claims**

1.  A computer-implemented method of matching atoms in a reactant system of atoms with corresponding atoms in a product system of atoms, said reactant system producing said product system in a chemical reaction, said method comprising:

a) matching at least one group of one or more atoms in said reactant system with at least one group of one or more atoms in said product system if said groups of one or more atoms are within regions of common topology; and
b) matching a plurality of other atoms in said reactant system with a plurality of other atoms in said product system based on similarity in geometry of said other atoms in said reactant system with geometry of said other atoms in said product system.

2. The method of claim 1 wherein the method comprises the steps of

xa) receiving data representing atom arrangement and bonding in said reactant and product systems;

a) matching a group of one or more atoms in said reactant system with a group of one or more atoms in said product system if said groups are within regions of common topology;

aa) repeating step a until no more groups within a region of common topology are found; and

b) matching remaining unmatched atoms based on similarity in geometry of said remaining atoms in said reactant system with geometry of said remaining atoms in said product system.

3. The method of claim 2 wherein when step a) is repeated, atoms matched in previous iterations of step a) are excluded from consideration.

4. The method of any of claims 1 to 3 wherein said regions of topology are **characterized by** determining connectivity values of atoms within said groups of one or more atoms and connectivity values for atoms adjacent to said atoms within said groups.

5. The method of any of claims 1 to 4 where said regions of common topology contain more atoms than said groups of one or more atoms.

6. The method of claim 5 wherein said regions of common topology extend at least one bonded atom beyond said groups of one or more atoms.

7. The method of any of claims 1 to 6 wherein said matching based on similarity in geometry is a match that results in minimum total deviation of atom positions relative to each other between said reactant system and said product system.

8. The method of any of claims 1 to 7 further comprising the step of a user providing input to match one or more atoms.

9. The method of claim 8 wherein at least one of said user matched atoms is used as a central starting point for finding a plurality of atoms surrounded by regions of common topology, wherein said plurality of atoms includes said at least one user matched atom.

10. The method of any of claims 1 to 9 further comprising the step of matching atoms that are unique in said reactant system with corresponding atoms in said product system, said step of matching unique atoms operating independent of user input.

11. The method of claim 10 wherein at least one of said unique atoms is used as a central starting point for finding a plurality of atoms surrounded by regions of common topology, wherein said plurality of atoms includes said at least one unique atom.

12. A computer-implemented system for matching atoms in a reactant system of atoms with corresponding atoms in a product system of atoms, said reactant system producing said product system in a chemical reaction, said system comprising:

a memory storing data representing atom arrangement and bonding in said reactant and product systems;
a topology matching module operative to match a group of one or more atoms in said reactant system with a group of one or more atoms in said product system if said groups are within regions of common topology; and
a geometry matching module operative to match atoms based on similarity in geometry of atoms in said reactant system with geometry of atoms in said product system.

13. The system of claim 12 wherein after said topology matching module operates to match groups of atoms within regions of common topology, said geometry matching module operates to match all remaining unmatched atoms.

14. The system of claim 12 or 13 further comprising a unique atom matching module operative to match atoms that are unique in said reactant system with corresponding atoms in said product system.

**15.** The system of claim 14 wherein said topology matching module uses atoms matched by said unique atom matching module as a central starting point for finding a plurality of atoms within regions of common topology.

**16.** The system of any of claims 12 to 15 further comprising a user interface allowing a user to manually match atoms in said reactant system with atoms in said product system.

**17.** The system of claim 16 wherein said topology matching module uses said manually matched atoms as a central starting point for finding a plurality of atoms within regions of common topology.

**18.** The system of any of claims 12 to 17 further comprising a user interface, wherein said user interface displays results of matching by said topology matching module and said geometry matching module.

**19.** The system of claim 18 wherein said user interface allows a user to modify said results of matching.

**20.** The system of any of claims 12 to 19 further comprising:

one or more additional geometry matching modules, each geometry matching module operative to match atoms based on similarity in geometry of atoms in said reactant system with geometry of atoms in said product system, each geometry matching module using a different geometric algorithm;
a geometric algorithm selection module operative to select at least one geometry matching module for use.

**21.** The system of claim 20 wherein said geometric algorithm selection module receives user input to select at least one geometry matching module for use.

**22.** The system of claim 20 wherein said geometric algorithm selection module operates independent of user input to select at least one geometry matching module for use.

**FIGURE 1**

```
┌─────────────────────────────┐
│ Receive  data  representing │
│ atom    arrangement    and  │    100
│ bonding                     │
└─────────────────────────────┘
              │
              ▼
┌─────────────────────────────┐
│ Match definite atoms        │
│                             │    110
│                             │
└─────────────────────────────┘
              │
              ▼
┌─────────────────────────────┐
│ Match   atoms   based   on  │
│ regions of common topology  │    120
│                             │
└─────────────────────────────┘
              │
              ▼
┌─────────────────────────────┐
│ Match   atoms   based   on  │
│ similarity in geometry      │    130
│                             │
└─────────────────────────────┘
              │
              ▼
        ┌───────────┐
        │    END    │
        └───────────┘
```

**FIGURE 2A**

**FIGURE 2B**

**FIGURE 2C**

**FIGURE 3**

```
        310
     ┌──────────┐
     │User input│  yes   ┌─────────────────────┐
     │matches?  │───────▶│Match atoms indicated│  320
     └──────────┘        │by user input        │
          │              └─────────────────────┘
          │no                      │
          │◀───────────────────────┘
          ▼
        330
     ┌──────────────┐
     │Unmatched     │  yes
     │unique atoms  │────────────┐
     │present?      │            ▼
     └──────────────┘      ┌──────────────────┐
          │               │Match unique atoms│  340
          │no             └──────────────────┘
          │                        │
          │◀───────────────────────┘
          ▼
       ┌─────┐
       │ END │  350
       └─────┘
```

**FIGURE 4**

**FIGURE 5**

Remaining unmatched atoms? — 500

no → END — 520

yes

Select geometric matching algorithm — 510

Match atoms based on similarity in geometry — 530

**FIGURE 6**

620

Unique    atom
matching module

610

User Interface

630

Topology
Matching Module

600

Reactant/Product
Memory

640

Geometric
algorithm selection
module

Geometry
matching module

650a

Geometry
matching module

650b

.
.
.